Europäisches Patentamt

European Patent Office

Office européen des brevets

(11) Veröffentlichungsnummer: **0 194 411**
A2

(12) **EUROPÄISCHE PATENTANMELDUNG**

(21) Anmeldenummer: **86100343.2**

(22) Anmeldetag: **13.01.86**

(51) Int. Cl.⁴: **F 16 L 37/00**

(30) Priorität: **14.02.85 DE 3505044**

(43) Veröffentlichungstag der Anmeldung:
**17.09.86 Patentblatt 86/38**

(84) Benannte Vertragsstaaten:
**AT BE CH FR GB IT LI NL SE**

(71) Anmelder: **MESSER GRIESHEIM GMBH**
**Hanauer Landstrasse 330**
**D-6000 Frankfurt/Main 1(DE)**

(72) Erfinder: **Donnerhack, Andreas, Dr.**
**Bismarckstrasse 4**
**D-4150 Krefeld(DE)**

(72) Erfinder: **Jankowski, Detlef**
**Kortumstrasse 127**
**D-4100 Duisburg 1(DE)**

(72) Erfinder: **Pfeil-Schneider, Kurt**
**Johannesfeld 29**
**D-4054 Nettertal(DE)**

(72) Erfinder: **Thoma, Klemens**
**Am Kleckers 22**
**D-4150 Krefeld-Hüls 29(DE)**

(72) Erfinder: **Volker, Wolfgang**
**Pastorsbusch 35**
**D-4154 Tönisvorst 1(DE)**

(54) **Schlauchendstück mit auswechselbarer Düse.**

(57) In der Kryotherapie wird ein kalter Gasstrom durch einen Schlauch mit auswechselbarer Düse auf den zu behandelnden Gelenken und Körperparien appliziert. Die Düse kann durch Schraub-, Bajonett- oder Rastverbindungen befestigt werden. Bei den tiefen Arbeitstemperaturen bis herab zu −180° C können diese Verbindungen aber Schwierigkeiten bereiten, indem sie schwer arretierbar werden oder sich während des Betriebes lockern. Um diese Schwierigkeiten zu vermeiden, werden sowohl im Schlauchendstück als auch in der Düse ringförmige Permanentmagnete (13,16) befestigt, die beim Aufsetzen der Düse deren Haftung im Schlauchendstück bewirken.

EP 0 194 411 A2

Croydon Printing Company Ltd.

— 1 —

MESSER GRIESHEIM GMBH                    MG 1500

Kennwort: Magnetische Düsenbefestigung          EM 1187 b

Erfinder: Dr.A.Donnerhack                Ordner:A
          D.Jankowski
          K.Pfeil-Schneider
          K.Thoma
          W.Volker

## Schlauchendstück mit auswechselbarer Düse

Die Erfindung betrifft ein Schlauchendstück mit auswechselbarer Düse nach dem Oberbegriff des Anspruches 1.

In der Kryotherapie zur Rheumabehandlung wird ein kalter
Gasstrom durch einen Behandlungsschlauch mit auswechselbarer Düse auf den zu behandelnden Gelenken und Körperpartien appliziert. Die Düse kann hierbei beispielsweise
durch Schraub-, Bajonett- oder Rastverbindungen befestigt
werden. Bei den tiefen Arbeitstemperaturen bis herab zu
-180° C können diese Verbindungen aber Schwierigkeiten bereiten, indem sie schwer arretiebar werden oder sich
während des Betriebes lockern.

Der Erfindung liegt die Aufgabe zugrunde, eine Verbindung
zwischen Behandlungsschlauch und Düse zu schaffen,

MG 1500

die leicht hergestellt und gelöst werden kann, dabei verschleißarm und sicher bei allen Arbeitstemperaturen, insbesondere tiefen Arbeitstemperaturen ist.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Eine vorteilhafte Weiterbildung der Erfindung ist im Unteranspruch angegeben.

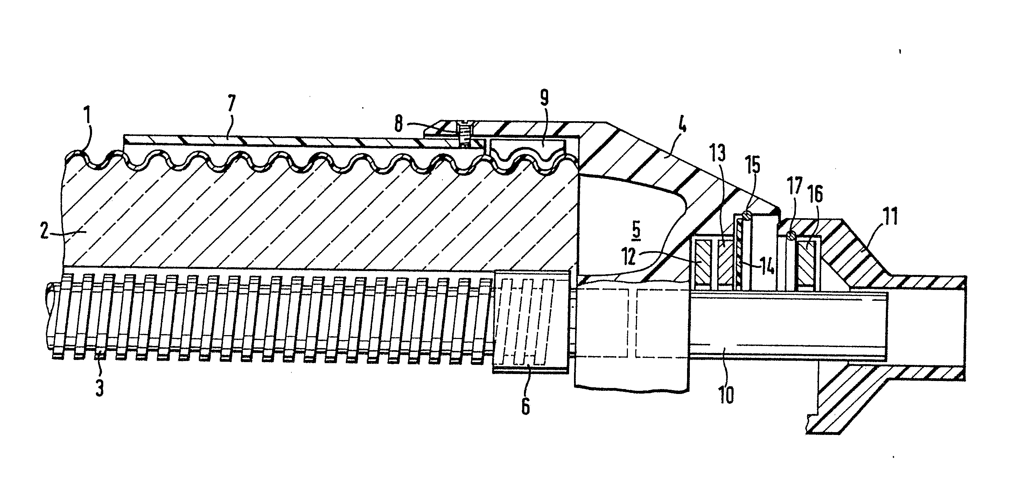

Die Zeichnung veranschaulicht ein Ausführungsbeispiel der Erfindung im Längsschnitt.

Der Behandlungsschlauch 1, bestehend aus einer Isolierung 2 und einem inneren Spiralgliederschlauch 3 ist in einem Schlauchendstück 4 befestigt. Das Schlauchendstück 4 besitzt eine Aussparung 5 zur Isolierung und Stabilisierung gegenüber thermischen Spannungen. Der Spiralgliederschlauch 3 ist mittels eines Gewindesübergangsstückes 6 im Schlauchendstück 4 arretiert. Der Behandlungsschlauch 1 ist mit einem Kunststoffrohr 7 in das Schlauchendstück 4 eingesetzt und durch drei über den Umfang verteilte Schrauben 8 mit diesem fest verbunden. Die Verbindung kann auch durch Verkleben erfolgen. Das Kunststoffrohr 7 stellt das eigentliche Griffrohr dar. Um die Einheit aus Kunststoffrohr 7 und Schlauchendstück 4 gegen den Behandlungsschlauch 1 zu arretieren, werden vor der Montage zwei Halbschalen 9 über den Behandlungsschlauch 1 gelegt, die über eine Breite von 2 bis 3 Gängen der äußeren Wellform des Schlauches angepaßt sind. Als Material für das Schlauchendstück eignet sich beispielsweise Polykarbonat. In das Schlauchendstück 4 ist ferner ein Rohrstück 10 eingeschraubt. Es dient zur Aufnahme der Düse 11. Um das Rohrstück 10 sind

MG 1500

in Aussparungen des Schlauchendstückes 4 ein Weicheisenring 12, ein Permanentmagnet 13 und eine Abdeckscheibe 14 aus Kunststoff angeordnet. Sie werden durch eine Ringfeder 15 gehalten. Auf entsprechende Weise wird in der Düse 11 ein Permanentmagnet 16 mittels der Ringfeder 17 gehalten. Der Permanentmagnet 16 bildet den Gegenpol zu dem Permanentmagneten 13. Erfindungsgemäß ermöglichen die ringförmigen Permanentmagnete 13 und 16 beim Zusammenführen von Schlauchendstück 4 und Düse 11 die Haftung beider Teile ineinander. Die Permanentmagnete 13 und 16 werden so angeordnet, daß kein seitlicher Spalt zwischen Schlauchendstück 4 und Düse 11 entsteht. Beim Abkühlen und der damit verbundenen Kontraktion des Schlauchendstückes 4 muß daher gewährleistet sein, daß die aufsteckbaren Düsen 11 immer entsprechend weit angezogen werden können.

Die Länge des Rohrstückes 10 wird so gewählt, daß der Kaltgasstrom im Spiralgliederschlauch 3 möglichst weit in die Düse 11 hineingeführt wird. Turbulenzen an nicht gasdichten Stellen werden dadurch vermieden. Die Düse 11 kann an ihrer Mündung durch Reduzier- bzw. Winkelstücke ergänzt werden. Bei der erfindungsgemäßen Art der Befestigung kann die Düse 11 leicht gedreht werden. Die Düse 11 besteht aus kältebeständigem Kunststoff. Überraschenderweise hat sich hierzu PVC als geeignet erwiesen, welches bei tiefen Temperaturen nur als beschränkt verwendungsfähig gilt.

Die Permanentmagnete 13 und 16 sowie die Dicke des Weicheisenringes 12 werden so aufeinander abgestimmt, daß die Haftung im Kontakt den gestellten Anforderungen genügt. Die Düse kann leicht manuell entfernt werden.

Die erfindungsgemäße Befestigung ermöglicht auch bei

0194411

MG 1500

tiefsten Arbeitstemperaturen eine einwandfreie Befestigung. Außerdem ist sie praktisch verschleißfrei, was für das in der Kryotherapie erforderliche häufige Auswechseln der Düsen von besonderem Vorteil ist. Der ringförmige Permanentmagnet 13 im Schlauchendstück 4 kann aus ringförmig angeordneten Stiftmagneten bestehen. Der Permanentmagnet 16 sollte jedoch auch in diesem Fall als massiver Ring ausgebildet sein. In Sonderfällen, d.h. bei ausreichender Stärke der Magnetkraft, kann auf den Permanentmagneten 13 ganz verzichtet werden; als Gegenstück genügt dann der Weicheisenring 12 allein.

Ba/Hi

0194411

5.Feb.1985

MG 1500

Patentansprüche

1. Schlauchendstück (4) mit auswechselbarer Düse (11)
zur Kaltgasbehandlung in der Kryotherapie,
dadurch gekennzeichnet,
daß das Schlauchendstück ein zentrales Rohrstück (10)
aufweist, auf welches die Düse aufschiebbar ist, und
daß sowohl im Schlauchendstück als auch in der Düse
ringförmige Permanentmagnete (13,16) befestigt sind,
die beim Aufsetzen der Düse deren Haftung im Schlauchendstück bewirken.

2. Schlauchendstück nach Anspruch 1,
dadurch gekennzeichnet,
daß der im Schlauchendstück befestigte ringförmige
Permanentmagnet (13) auf einem Weicheisenring (12)
aufliegt.

Ba/Hi